# EUROPÄISCHE PATENTSCHRIFT

(11) **EP 0 446 431 B1**
(45) Veröffentlichungstag und Bekanntmachung des Hinweises auf die Patenterteilung: **27.09.1995**
(21) Anmeldenummer: 90122814.8
(22) Anmeldetag: 29.11.1990
(51) Int. Cl.: B32B 27/12

(54) **Trägermaterial für medizinische Zwecke**
Carrier for medical uses
Support à usage médical

(30) Priorität: 13.03.1990 DE 4007891
(43) Veröffentlichungstag der Anmeldung: 18.09.1991
(73) Patentinhaber: Beiersdorf Aktiengesellschaft, 20245 Hamburg (DE)
(72) Erfinder: Götz, Gabriela, W-2000 Hamburg 65 (DE); Schulte, Dietrich, Dr., W-2080 Pinneberg (DE); Eggers, Ursula, W-2000 Hamburg 52 (DE)

(56) Entgegenhaltungen:
- EP-A- 0 006 264
- EP-A- 0 257 133
- WO-A-89/01345
- US-A- 4 727 868

## Beschreibung

Die Erfindung betrifft ein Trägermaterial für medizinische Zwecke, vorzugsweise Pflaster und Wundverbände, das aus einem mehrschichtigen Laminat besteht und sauerstoff- und wasserdampfdurchlässig sowie keimdicht, d.h. dicht gegen das Eindringen von Bakterien, ist.

Als Trägermaterialien für medizinische Zwecke, d.h. in erster Linie für Pflaster und Wundverbände oder zur Fixierung von z.B. Injektionskanülen, sind bereits zahlreiche Materialien auf Folien-, Gewebe, Gewirke oder Vlies-Basis bekannt und werden auch in der Praxis eingesetzt. Da die Produkte in der Regel eine ganze Reihe von Anforderungen erfüllen müssen, indem sie zwar weich und schmiegsam sein sollen, je nach Verwendungszweck bevorzugt mit "textilem Griff", aber auch genügend stabil und widerstandsfähig sein müssen, um den Wundbereich genügend zu schützen, wurden auch schon die verschiedensten mehrschichtigen Produkte vorgeschlagen. Derartige Laminate sind beispielsweise in den EP-A 45 592, 81 990, 257 133, WO 85/05322, 85/05373, 89/01345, US-PS 4 727 868, 4 751 133 beschrieben. Sie werden hergestellt durch Tauchen oder Verbinden der einzelnen Trägermaterialien über eine Klebschicht und weisen dabei den Nachteil auf, daß entweder der angenehme "textile Griff" verloren geht, wenn beispielsweise beim Tauchen das Fasergewebe ganz von Polymermasse umgeben wird, wie bei der US-A-4 727 868, oder daß sie durch das Zusammenkaschieren mit Klebmassenschichten einen großen Teil ihrer Flexibilität und Geschmeidigkeit verlieren.

Bei einem Wundverband gemäß der EP-A 257 133 ist beispielsweise eine Folie über eine Klebmassenschicht mit einer textilen Schicht verbunden, wobei zusätzlich eine stark absorptionsfähige hydrophile Schicht in der textilen Schicht eingebettet ist.

Auch in der WO 89/01345 ist ein vielschichtiger Verband beschrieben, bei dem sich zwischen der Folie und dem Vlies eine Klebschicht und außerdem eine klebstoffabweisende Schicht befindet, um die beiden Schichten wieder leicht voneinander trennen zu können.

Derartige Verbandmaterialien sind in der Herstellung vergleichsweise kompliziert und teuer aufgrund der zahlreichen Arbeitsgänge. Außerdem verdickt und versteift jede zusätzliche Schicht die Laminate, so daß sie sich weniger gut der Haut anschmiegen können.

Aufgabe der Erfindung war es deshalb, ein Trägermaterial zu schaffen, das sich für medizinische Pflaster eignet und die vorgenannten Nachteile nicht aufweist, sondern sowohl geschmeidig und flexibel als auch gut handhabbar ohne zusätzlichen Hilfsträger sowie sauerstoff- und wasserdampfdurchlässig aber undurchlässig für Wasser und Bakterien ist.

Gelöst wird diese Aufgabe durch ein Trägermaterial gemäß Anspruch 1, wobei die drei Schichten zusammen sauerstoff- und wasserdampfdurchlässig jedoch keimdicht sind.

Die polymeren Filmschichten bestehen vorzugsweise aus elastischen, thermoplastischem Polyurethanen, wie sie in der DE-C 19 34 710 beschrieben sind und die sich durch eine gute Hautverträglichkeit sowie Sauerstoff- und Wasserdampfdurchlässigkeit auszeichnen. Besonders vorteilhaft haben sich aliphatische Polyesterurethane erwiesen.

Daneben lassen sich jedoch auch Filme auf anderer Grundlage wie z.B. Acrylat-Copolymere oder die verschiedensten Block-Copolymere und deren Mischungen mit anderen Basispolymeren, wie sie beispielsweise in den PCT-Anmeldungen WO 85/05373 und WO 85/05322 aufgeführt sind, verwenden.

Die beiden Filmschichten, die vorzugsweise eine Dicke von jeweils etwa 0,01 bis 0,035 mm, besonders bevorzugt etwa 0,015 bis 0,025 mm, aufweisen, können aus den gleichen Polymeren bestehen oder auch aus verschiedenen, sofern diese derart miteinander verträglich sind, daß eine Haftung aufeinander möglich ist. üblicherweise sind sie farblos und etwas opak, sie können jedoch auch z.B. hautfarben oder weiß eingefärbt sein.

Wesentlich ist, daß sich aus den synthetischen Polymeren kontinuierliche Filme durch Gießen, Rakeln, Extrudieren oder andere bekannte Methoden zur Filmbildung herstellen lassen, die Filme hautverträglich, flexibel und bis zu einem gewissen Grade elastisch sind sowie eine gute Sauerstoff- und Wasserdampfdurchlässigkeit aufweisen. Letztere sollte mindestens 500g/m²/24h/38^{o}C bei 95% Feuchtigkeitsgefälle (DAB9) betragen.

Als textile Schichtmalterialien eignen sich insbesondere ein Lochmuster aufweisende Vliese, wie z.B. das bekannte Sontara^{R}-Vlies, welches ohne Bindemittel nach dem sog. "spunlaced"-Verfahren hergestellt ist und aus Polyesterfasern besteht. Sein mittlerer Porendurchmesser beträgt je nach Muster vorzugsweise ca. 0,2 bis 1,0 mm.

Bei dem sog. "spunlaced"-Verfahren wird das Rohvlies ohne jegliche Anwendung von chemischen Bindemitteln durch eine Vielzahl von Wasserstrahlen aus einem feinen Düsensystem in Verbindung mit einer geeigneten Lochmaske zur Formgebung, z.B. Lochbildung oder Musterung, durch Verhaken der Einzelfasern verfestigt. Auf diese Weise entstehen Vliese mit textilem Griff und großer Porösität.

Auch feiner Gittertüll aus beispielsweise Polyester und mit einem mittleren Porendurchmesser von vorzugsweise 1,0 bis 3,0 mm hat sich als geeignet erwiesen.

Aber auch entsprechende Gewebe oder Gewirke können eingesetzt werden.

Wichtig ist, daß die textile Schicht aus einem Material besteht, das weich, biegsam und zumindest in einer Richtung elastisch oder dehnbar ist, wobei diese Nachgiebigkeit ungefähr der Nachgiebigkeit des Folienmaterials entsprechen soll, um sich dadurch besonders gut den Bewegungen der Haut anpassen zu können.

Ein weiteres wichtiges Merkmal dieser Schicht sind die relativ großen Poren oder öffnungen, damit bei der Herstellung die Polymersubstanz der zweiten Filmschicht, die sich in noch etwas flüssigem Zustand befindet, wenn das textile Material aufgebracht wird, in die Poren eindringen kann. Das Vlies oder der Tüll versinken dadurch mehr oder weniger tief in der Polymerschicht und werden in dieser Schicht verankert, so daß ein festes Verbundprodukt entsteht.

Werden die Schichtstärken oder Viskositäten derart gewählt, daß das textile Produkt nur oberflächlich einsinkt, bleibt sein erwünschter angenehmer "textiler Griff" erhalten.

Das erfindungsgemäße Trägermaterial kann auf einer der beiden Seiten, vorzugsweise auf der Textil-Seite, in bekannter Weise mit einer hautverträglichen Selbstklebeschicht versehen sein, wobei diese mikroporös ausgebildet sein kann, um eine gute Sauerstoff- und Wasserdampfdurchlässigkeit für das ganze Produkt zu gewährleisten.

Ferner kann mittig auf der selbstklebenden Seite eine Wundauflage angeordnet sein, wobei noch ein ausreichend breiter klebender Rand frei bleiben sollte, wenn das Trägermaterial als Wundverband eingesetzt werden soll.

Die Selbstklebeschicht und die gegebenenfalls auf ihr angeordnete Wundauflage sind üblicherweise mit einem klebstoffabweisend ausgerüsteten Hilfsträger, meist aus silikonisiertem Papier, bis zur Verwendung abgedeckt.

Die Herstellung des erfindungsgemäßen Trägermaterials wird vorzugsweise in der Weise durchgeführt, daß zuerst die erste Filmschicht auf einem Hilfsträger erzeugt wird, indem das Polymer oder Copolymer als Lösung, Dispersion oder Schmelze in dünner Schicht aufgebracht und getrocknet wird. Anschließend wird in gleicher Weise die zweite dünne Filmschicht aus dem gleichen oder einem mit dem Polymer der ersten Schicht verträglichen zweiten Polymer oder Copolymer auf der ersten Filmschicht hergestellt jedoch nicht vollkommen getrocknet bzw. abgekühlt. Dann wird auf die noch fließfähige zweite Filmschicht das textile Material aufgebracht (aufkaschiert) und das Produkt fertig verfestigt, d.h. getrocknet bzw. abgekühlt.

Das Laminat wird vorzugsweise mit einer Selbstklebemasse beschichtet indem die Klebemasse, vorzugsweise als Lösung, auf einen klebstoffabweisend ausgerüsteten Zwischenträger, beispielsweise silikonisiertes Kraftpapier oder silikonisierte Folie, ausgestrichen und durch schnelles Verdampfen des Lösungsmittels bei erhöhter Temperatur zu einem feinblasigen Zustand getrocknet wird. Nach Abkühlen auf Raumtemperatur wird das Laminat auf die Klebeschicht aufkaschiert und gegebenenfalls einem Kalandervorgang gemäß DBP 15 69 901 unterworfen. Dadurch wird die Klebemasse auf das Trägermaterial übertragen und die feinen Bläschen werden aufgebrochen, wodurch ein feinporiges mikroporöses Gefüge entsteht. Wenn es die Klebemasse erfordert, kann noch ein Vernetzungsprozeß durch Wärmeeinwirkung oder andere Verfahren wie UV- oder Elektronen-Bestrahlung nachgeschaltet werden.

Anschließend wird das Produkt für den Gebrauch konfektioniert, d.h. die Hilfsträger werden entfernt, das Material wird in Streifen gewünschter Länge und Breite geschnitten, diese werden gegebenenfalls mit je einer mittigen Wundauflage versehen sowie auf der Klebschichtseite mit einem silikonisierten Abdeckpapier eingedeckt, die einzelnen Pflaster werden in eine Verpackung eingesiegelt und durch γ-Bestrahlung sterilisiert.

Das auf diese Weise hergestellte erfindungsgemäße Trägermaterial aus dem dreischichtigen Laminat erfüllt alle von der Praxis gestellte Anforderungen. Es ist so flexibel und dehnungsfähig, daß es sich den Unebenheiten und Bewegungen der Haut anpassen kann, und wasserdampf- und sauerstoffdurchlässig, so daß die Haut darunter "atmen" kann, gleichzeitig ist es aber dicht gegen das Eindringen von Bakterien, da Mikroporen in der ersten Schicht durch die zweite Beschichtung verschlossen werden, und außerdem durch die eingefügte poröse Textilschicht weich und so stabil, daß es ohne Hilfsträger benutzt und gehandhabt werden kann.

Die folgenden Beispiele sollen die Herstellung der neuen Pflaster näher erläutern.

### Beispiel 1

Auf einem Gießpapier, d.h. ein sog. "Casting Paper" (z.B. "Transkote" der Fa. Scott Continental) mit matter Oberfläche wird eine Polyurethan-Lösung aus einem Einkomponenten-Polyesterurethan auf Basis eines aliphatischen Diisocyanats (Impranil^{R} der Fa. Bayer) in einem Lösungsmittelgemisch aus Isopropanol, Toluol und Ethylglykol ausgestrichen in einer Dicke von etwa 15-20g/m² (bezogen auf den Feststoff), was einer Dicke von etwa 0,015 - 0,020 mm (entspr. 15 - 20 µm) entspricht, bei ca. 90^{o}C blasenfrei getrocknet und so die erste Filmschicht hergestellt. Dann wird auf diesen Film die zweite Filmschicht in gleicher Weise und Dicke ausgestrichen. In die noch nasse Schicht wird ein Polyestervlies mit Lochstruktur (Sontara^{R}, Flächengewicht ca. 45g/m², Dicke ca. 0,50 mm) eingelegt und wieder bei ca. 90^{o}C getrocknet.

Das fertige Laminat wird wie folgt mit einer mikroporösen Selbstklebeschicht versehen:
Auf einem silikonisierten Zwischenträger (Papier oder Folie) wird eine Selbstklebemasse, die durch Copolymerisation von 49 Gewichtsteilen 2-Äthylhexylacrylat, 49 Gewichtsteilen n-Butylacrylat und 2 Gewichtsteilen Glycidylmethacrylat erhalten wurde, aus einer Lösung in einem Aceton-Benzin-Gemisch in einer solchen Menge aufgetragen, daß nach dem Trocknen eine Schichtstärke von etwa 50 g/m² erzielt wird. Um das Lösungsmittelgemisch rasch abzudunsten, wird das mit der Klebemasse beschichtete Trennpapier anschließend durch einen stufenweise beheizten Trockenkanal mit Temperaturen von 60-100^{o}C geleitet, wodurch sich in der Klebeschicht eine Vielzahl winziger Blaschen bildet. Nach Abkühlung auf Raumtemperatur wird die getrocknete Klebeschicht auf die Vliesseite des Laminats aufkaschiert.

Danach wird das Verbundprodukt bei einem Druck von etwa 5 kp/cm² kalandert. Nach diesem Kalandervorgang, bei dem die kleinen Bläschen aufbrechen und gleichzeitig die Klebeschicht sich fest mit dem Laminat verbindet, werden die breiten Rollen zu schmalen Rollen unterschiedlicher Breiten aufgeschnitten, wird das noch anhaftende Trennpapier abgezogen, die geschnittenen Bänder zu Einzelpflastern mit Wundauflage weiter verarbeitet und erneut mit silikonisiertem Trennpapier eingedeckt. Nach dem Verpacken werden die fertigen Pflaster einer γ-Bestrahlung (2,5-Mrad absorbierte Dosis) unterworfen und auf diese Weise sterilisiert. Gleichzeitig erfährt die Klebemasse eine Nachvernetzung, die ihre Kohäsion in wünschenswerter Weise verbessert.

### Beispiel 2

In gleicher Weise, wie in Beispiel 1 beschrieben, wird das Zwischenprodukt aus den beiden Polyurethan-Filmschichten hergestellt und dann in die noch nasse zweite Schicht ein feiner Gitter-Tüll aus Polyester (sechseckige-Wabenstruktur, m²-Gewicht ca. 26g/m², Dicke ca. 0,17 mm, Lochzahl ca. 31 p/inch², mittlerer Durchmesser der Löcher 1,5-2 mm) eingelegt und getrocknet.

Das fertige Laminat wird mit einer UV-vernetzbaren Selbstklebemassenschicht gemäß DBP 27 43 979 versehen. Dabei wird im wesentlichen verfahren wie im vorstehenden Beispiel 1 ausgeführt, jedoch ein Copolymerisat aus 73,59 Gew.-%, 2-Äthylhexylacrylat, 20 Gew.-% Butylacrylat, 6 Gew.-% Acrylsäure und 0,41 Gew.-% Benzoinacrylat eingesetzt.

Nach dem Zusammenkaschieren des mit der getrockneten Selbstklebemasse beschichteten Zwischenträgers mit dem Laminat wird das Verbundprodukt etwa 1 sec. lang mit 4 x 11-12 kW UV-Strahlung bestrahlt, um die Klebemasse zu vernetzen. Die Weiterverarbeitung und Konfektionierung erfolgt analog Beispiel 1. Das Produkt zeigt eine besonders gute Anfaßklebrigkeit (tack).

## Patentansprüche

1. Trägermaterial für medizinische Pflaster bestehend aus einem Laminat aus einer ersten polymeren Filmschicht, einer zweiten, auf der ersten Schicht erzeugten polymeren Filmschicht und einer dritten, in der zweiten Schicht teilweise eingebetteten und auf diese Weise darin verankerten Schicht aus einem makroporösen textilen Material.

2. Trägermaterial nach Anspruch 1, dadurch gekennzeichnet , daß die erste und die zweite Filmschicht eine Dicke von jeweils etwa 0,01 bis 0,035 mm aufweisen.

3. Trägermaterial nach Anspruch 1, dadurch gekennzeichnet, daß die erste und zweite Filmschicht aus einem elastischen Polyurethan bestehen.

4. Trägermaterial nach Anspruch 1, dadurch gekennzeichnet, daß die textile Schicht aus einem querelastischen, nach dem "spunlaced"-Verfahren hergestellten Polyestervlies besteht.

5. Trägermaterial nach Anspruch 4, dadurch gekennzeichnet, daß der mittlere Porendurchmesser in dem Faservlies ca. 0,2 bis 1,5 mm beträgt.

6. Trägermaterial nach Anspruch 1, dadurch gekennzeichnet, daß die textile Schicht aus einem Gittertüll besteht.

7. Trägermaterial nach Anspruch 6, dadurch gekennzeichnet, daß der Gittertüll aus Polyesterfasern besteht und einen mittleren Porendurchmesser von ca. 1,0 - 3,0 mm aufweist.

8. Trägermaterial nach Anspruch 1, dadurch gekennzeichnet, daß es auf der textilen Materialseite mit einer hautverträglichen Selbstklebemasse beschichtet ist.

9. Trägermaterial nach Anspruch 8, dadurch gekennzeichnet, daß auf der Selbstklebeschicht eine Wundauflage angeordnet ist, welche kleiner als die Trägermaterialfläche ist.

10. Verfahren zur Herstellung eines Trägermaterials für medizinische Pflaster gemäß einem der Ansprüche 1 bis 7, indem die erste Filmschicht auf einem Hilfsträger erzeugt wird, die zweite Filmschicht danach auf der ersten Schicht hergestellt wird, anschließend das textile Material auf die noch nicht verfestigte zweite Filmschicht aufgebracht wird, dann das Laminat fertig verfestigt wird und ein Trägermaterial gemäß den Ansprüchen 1 bis 7 erhalten wird.

## Claims

1. Carrier material for medical plasters comprising a laminate of a first polymeric film layer, a second polymeric film layer produced on the first layer and a third layer of a macroporous textile material partly embedded in the second layer and anchored therein in this manner.

2. Carrier material according to Claim 1, characterized in that the first and the second film layer have a thickness of in each case about 0.01 to 0.035 mm.

3. Carrier material according to Claim 1, characterized in that the first and the second film layer comprise an elastic polyurethane.

4. Carrier material according to Claim 1, characterized in that the textile layer comprises a transversely elastic polyester nonwoven prepared by the "spunlaced" process.

5. Carrier material according to Claim 4, characterized in that the average pore diameter in the fibrous non-woven is about 0.2 to 1.5 mm.

6. Carrier material according to Claim 1, characterized in that the textile layer comprises a lattice tulle.

7. Carrier material according to Claim 6, characterized in that the lattice tulle comprises polyester fibres and has an average pore diameter of about 1.0-3.0 mm.

8. Carrier material according to Claim 1, characterized in that it is coated on the textile material side with a self-adhesive composition tolerated by the skin.

9. Carrier material according to Claim 8, characterized in that a wound pad which is smaller than the carrier material area is arranged on the self-adhesive layer.

10. Process for the preparation of a carrier material for medical plasters according to one of Claims 1 to 7, in which the first film layer is produced on an auxiliary carrier, the second film layer is then prepared on the first layer, and the textile material is then applied to the second film layer, which has not yet solidified, solidification of the laminate is then completed and a carrier material according to Claims 1 to 7 is obtained.

## Revendications

1. Support pour pansements à usage médical, se composant d'un produit laminé fait d'une première couche de film polymère, d'une deuxième couche de film polymère produite sur la première couche et d'une troisième couche faite en matériau textile à macroporosité, enrobée en partie dans la deuxième couche et ancrée dans celle-ci de cette manière.

2. Support selon la revendication 1, caractérisé en ce que la première couche de film et la deuxième couche de film présentent une épaisseur à chaque fois d'environ 0,01 à 0,035 mm.

3. Support selon la revendication 1, caractérisé en ce que la première couche de film et la deuxième couche de film se composent d'un polyuréthane élastique.

4. Support selon la revendication 1, caractérisé en ce que la couche textile se compose d'un non-tissé en polyester, à élasticité transversale, fabriqué conformément au procédé "spunlaced" [texturé par filage].

5. Support selon la revendication 4, caractérisé en ce que le diamètre moyen de pores dans le non-tissé à fibres est d'environ 0,2 à 1,5 mm.

6. Support selon la revendication 1, caractérisé en ce que la couche textile se compose d'une tulle à réseau.

7. Support selon la revendication 6, caractérisé en ce que la tulle à réseau se compose de fibres de polyester et présente un diamètre moyen de pores d'environ 1,0 - 3,0 mm.

8. Support selon la revendication 1, caractérisé en ce qu'il est enduit, du côté du matériau textile, d'une masse auto-adhésive compatible avec la peau.

9. Support selon la revendication 8, caractérisé en ce qu'est disposée, sur la couche auto-adhésive, une zone d'appui de lésion, qui est inférieure à la surface du support.

10. Procédé de fabrication d'un support pour pansements à usage médical conformément à l'une quelconque des revendications 1 à 7, en ce que l'on réalise la première couche de film sur un support auxiliaire, en ce que l'on prépare ensuite la deuxième couche de film sur la première couche, en ce que l'on applique subséquemment le matériau textile sur la deuxième couche de film qui n'est pas encore solidifiée, en ce que l'on complète alors la solidification du produit laminé et en ce que l'on obtient un support conformément aux revendications 1 à 7.
